# EUROPEAN PATENT APPLICATION

(11) **EP 4 410 777 A1**
(43) Date of publication of application: **07.08.2024**
(21) Application number: 22872243.5
(22) Date of filing: 27.09.2022
(51) Int. Cl.: C07D 231/56, C07D 401/14, C07D 403/14, A61K 31/416, A61K 31/44, A61K 31/445, A61K 31/506, A61P 35/00

(54) **COMBINED PHARMACEUTICAL COMPOSITION OF CDK4/6 INHIBITOR AND AROMATASE INHIBITOR**

(30) Priority: 27.09.2021 CN 202111133804
(71) Applicant: CHIA TAI TIANQING PHARMACEUTICAL GROUP CO., LTD., Lianyungang, Jiangsu 222062 (CN)
(72) Inventor: FENG, Fan, Lianyungang, Jiangsu 222062 (CN); YU, Ding, Lianyungang, Jiangsu 222062 (CN); ZHANG, Xiquan, Lianyungang, Jiangsu 222062 (CN); WANG, Yueting, Lianyungang, Jiangsu 222062 (CN)
(74) Representative: WSL Patentanwälte Partnerschaft mbB
(86) International application number: PCT/CN2022/121752
(87) International publication number: WO 2023/046200

(57) **Abstract**

The present disclosure relates to a combined pharmaceutical composition of a CDK4/6 inhibitor and an aromatase inhibitor, and the use thereof in the treatment of breast cancer. Compared with separate administration of any agent in the composition, the combined pharmaceutical composition of the present disclosure yields a better therapeutic effect on slowing tumor growth or even eliminating tumors.

## Description

### CROSS-REFERENCE TO RELATED APPLICATIONS

The present application claims priority and benefit to the Chinese Patent No. 202111133804.5 filed with the National Intellectual Property Administration, PRC on September 27, 2021, which is incorporated herein by reference in its entirety.

### TECHNICAL FIELD

The present disclosure belongs to the technical field of medicines, and relates to a combined pharmaceutical composition of a CDK4/6 inhibitor and an aromatase inhibitor, and use thereof for treating breast cancer.

### BACKGROUND

Cyclin-dependent kinase (CDK) 4/6 is a key regulator of cell cycle and is capable of triggering the cell cycle from the growth phase (G1 phase) to the DNA replication phase (S1 phase). In the process of cell proliferation, the complex formed by CDK4/6 and cyclin D can phosphorylate the retinoblastoma protein (Rb). Once the tumor suppressor protein Rb is phosphorylated, it can release transcription factor E2F to which it tightly binds in the unphosphorylated state. E2F activates further transcription to promote the cell cycle through the restriction point (R point) from G1 phase to S phase. Therefore, the inhibition of CDK4/6 and thus its inability to form cyclin D-CDK4/6 complex, can block the progression of cell cycle from G1 phase to S phase, thereby achieving the purpose of inhibiting tumor proliferation. A substituted 2-hydrogen-pyrazole derivative as a selective CDK4/6 inhibitor is disclosed in WO2016141881, and a compound of formula (I) with the following structure is also specifically disclosed:

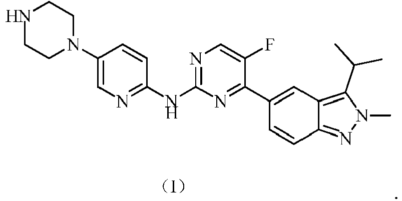

The worldwide cancer report data shows about 1.7 million new cases of breast cancer and about 0.5 million deaths worldwide in 2012, which respectively account for 25% of all new cancers and 15% of all cancer deaths in women and both rank first. In new breast cancer cases each year, 3%-10% of women have distant metastasis at the time of diagnosis. 30%-40% of patients with early breast cancer may develop advanced breast cancer with a 5-year survival rate of about 20%.

Patients with advanced breast cancer (ABC) are special in the selection and efficacy of treatment regimens. Currently, there is still a lack of a recognized standard treatment regimen. The overall median survival time for advanced breast cancer is 2-3 years, varying between different molecular subtypes. For ABC patients positive for human epidermal growth factor receptor 2 (HER2), anti-HER2 treatment may alter the natural course of HER2-positive ABC patients and significantly prolong survival time; however, for triple-negative ABC patients, the overall prognosis has not improved significantly. Hormone receptor-positive (HR+) breast cancer accounts for about 65%-75% of breast cancer, and endocrine therapy is the first choice for hormone receptor-positive (HR+) metastatic breast cancer due to its equivalent efficacy and decreased toxic and side effects compared with chemotherapy.

Although endocrine therapy is the main treatment regimen of hormone receptor-positive breast cancer, about 30% of hormone receptor-positive breast cancer has primary drug resistance to endocrine therapy, and almost all patients have secondary drug resistance in follow-up treatment. Therefore, how to overcome the endocrine therapy resistance is a problem to be solved in the field of breast cancer treatment.

### SUMMARY

In one aspect, the present disclosure provides a combined pharmaceutical composition comprising a compound of formula (I) or a pharmaceutically acceptable salt thereof and an aromatase inhibitor:

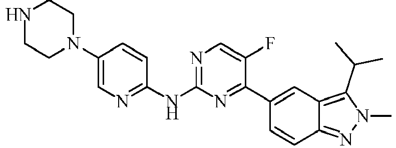

In another aspect, the present disclosure provides a combined pharmaceutical composition for use in treating or preventing breast cancer, comprising a compound of formula (I) or a pharmaceutically acceptable salt thereof and an aromatase inhibitor. Alternatively, the present disclosure provides a method for treating or preventing breast cancer, comprising administering to a subject in need thereof the combined pharmaceutical composition described above. Alternatively, the present disclosure provides use of the combined pharmaceutical composition described above for preparing a medicament or kit for treating or preventing breast cancer. Alternatively, the present disclosure provides a method of administration, comprising administering to a subject in need thereof the combined pharmaceutical composition described above.

In some embodiments of the present disclosure, the compound of formula (I) or the pharmaceutically acceptable salt thereof and the aromatase inhibitor in the combined pharmaceutical composition are packaged in a kit, and the kit further comprises an instruction for use of the compound of formula (I) or the pharmaceutically acceptable salt thereof in combination with the aromatase inhibitor for treating or preventing breast cancer.

In some embodiments of the present disclosure, the compound of formula (I) or the pharmaceutically acceptable salt thereof and the aromatase inhibitor in the combined pharmaceutical composition are packaged separately in their respective kits, and the kits further comprise instructions for use of the compound of formula (I) or the pharmaceutically acceptable salt thereof in combination with the aromatase inhibitor for treating or preventing breast cancer.

In some embodiments of the present disclosure, the compound of formula (I) or the pharmaceutically acceptable salt thereof and the aromatase inhibitor in the combined pharmaceutical composition are each in the form of a pharmaceutical composition and can be administered simultaneously, sequentially, or at intervals.

In some embodiments of the present disclosure, the combined pharmaceutical composition comprises a pharmaceutical composition of the compound of formula (I) or the pharmaceutically acceptable salt thereof, and a pharmaceutical composition of the aromatase inhibitor.

In some embodiments of the present disclosure, the pharmaceutical composition of the compound of formula (I) or the pharmaceutically acceptable salt thereof and the pharmaceutical composition of the aromatase inhibitor in the combined pharmaceutical composition are packaged in a kit, and the kit further comprises an instruction for use of the pharmaceutical composition of the compound of formula (I) or the pharmaceutically acceptable salt thereof in combination with the pharmaceutical composition of the aromatase inhibitor for treating or preventing breast cancer.

In some embodiments of the present disclosure, the pharmaceutical composition of the compound of formula (I) or the pharmaceutically acceptable salt thereof and the pharmaceutical composition of the aromatase inhibitor in the combined pharmaceutical composition are packaged separately in their respective kits, and the kits further comprise instructions for use of the pharmaceutical composition of the compound of formula (I) or the pharmaceutically acceptable salt thereof in combination with the pharmaceutical composition of the aromatase inhibitor for treating or preventing breast cancer.

In some embodiments of the present disclosure, in the combined pharmaceutical composition, the pharmaceutically acceptable salt of the compound of formula (I) is a maleate, such as a monomaleate of the compound of formula (I).

In some embodiments of the present disclosure, the aromatase inhibitor is selected from the group consisting of letrozole and anastrozole.

In some embodiments of the present disclosure, the combined pharmaceutical composition comprises 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I), or a pharmaceutical composition thereof.

In some embodiments of the present disclosure, the combined pharmaceutical composition comprises 20 mg, 40 mg, 60 mg, 80 mg, 100 mg, 120 mg, 150 mg, 180 mg, or 240 mg of the compound of formula (I) or the pharmaceutically acceptable salt thereof based on the compound of formula (I), or a pharmaceutical composition thereof.

In some embodiments of the present disclosure, the combined pharmaceutical composition comprises 60 mg, 120 mg, or 180 mg of the compound of formula (I) or the pharmaceutically acceptable salt thereof based on the compound of formula (I), or a pharmaceutical composition thereof.

In some embodiments of the present disclosure, in the combined pharmaceutical composition, the pharmaceutical composition of the compound of formula (I) or the pharmaceutically acceptable salt thereof is in a single-dose form or in a multi-dose form. In some embodiments of the present disclosure, in the combined pharmaceutical composition, the pharmaceutical composition of the compound of formula (I) or the pharmaceutically acceptable salt thereof is in a multi-dose form.

In some embodiments of the present disclosure, in the combined pharmaceutical composition, the content of the compound of formula (I) or the pharmaceutically acceptable salt thereof is a daily dose or a one-day dose.

In some embodiments of the present disclosure, in the combined pharmaceutical composition, the content of the compound of formula (I) or the pharmaceutically acceptable salt thereof is a once-daily dose or a once-a-day dose. In some embodiments of the present disclosure, in the combined pharmaceutical composition, the content of the compound of formula (I) or the pharmaceutically acceptable salt thereof is a once-daily dose or a once-a-day dose, with each dose being a single dose or multiple doses, preferably multiple doses.

In some embodiments of the present disclosure, the combined pharmaceutical composition comprises a pharmaceutical composition of the compound of formula (I) or the pharmaceutically acceptable salt thereof in a single dose of 50 mg, 60 mg, 120 mg, or 180 mg, based on the compound of formula (I). Alternatively, the combined pharmaceutical composition is in the form of a single-administration formulation, and the combined pharmaceutical composition comprises 50 mg, 60 mg, 120 mg, or 180 mg of the compound of formula (I) or the pharmaceutically acceptable salt thereof based on the compound of formula (I), or a pharmaceutical composition thereof.

In some embodiments of the present disclosure, the combined pharmaceutical composition comprises a pharmaceutical composition of the compound of formula (I) or the pharmaceutically acceptable salt thereof in multiple doses of 50 mg, 60 mg, 120 mg, or 180 mg, based on the compound of formula (I). Alternatively, the combined pharmaceutical composition is in the form of a single-administration formulation, with each dose being a multiple-dose, and the combined pharmaceutical composition comprises 50 mg, 60 mg, 120 mg, or 180 mg of the compound of formula (I) or the pharmaceutically acceptable salt thereof based on the compound of formula (I), or a pharmaceutical composition thereof.

In some embodiments of the present disclosure, the combined pharmaceutical composition comprises a pharmaceutical composition of the compound of formula (I) or the pharmaceutically acceptable salt thereof in a single dose of 50 mg or 60 mg, based on the compound of formula (I). Alternatively, the combined pharmaceutical composition is in the form of a single-administration formulation, and the combined pharmaceutical composition comprises 50 mg or 60 mg of the compound of formula (I) or the pharmaceutically acceptable salt thereof based on the compound of formula (I), or a pharmaceutical composition thereof.

In some embodiments of the present disclosure, the combined pharmaceutical composition comprises a pharmaceutical composition of the compound of formula (I) or the pharmaceutically acceptable salt thereof in a single dose of 60 mg, based on the compound of formula (I). Alternatively, the combined pharmaceutical composition is in the form of a single-administration formulation, and the combined pharmaceutical composition comprises 60 mg of the compound of formula (I) or the pharmaceutically acceptable salt thereof based on the compound of formula (I), or a pharmaceutical composition thereof.

In some embodiments of the present disclosure, the pharmaceutical composition of the compound of formula (I) or the pharmaceutically acceptable salt thereof comprised in the combined pharmaceutical composition is packaged in a kit, and the kit further comprises an instruction for use of the compound of formula (I) or the pharmaceutically acceptable salt thereof for treating or preventing breast cancer.

### Combined pharmaceutical composition of compound of formula (I) or pharmaceutically acceptable salt thereof and letrozole

In some embodiments of the present disclosure, in the combined pharmaceutical composition, the aromatase inhibitor is letrozole.

In some embodiments of the present disclosure, the combined pharmaceutical composition comprises 2.5-12.5 mg, 2.5-10 mg, 2.5-7.5 mg, or 2.5-5 mg of letrozole or a pharmaceutical composition thereof. Alternatively, the combined pharmaceutical composition is in the form of a single-administration formulation, and the combined pharmaceutical composition comprises 2.5-12.5 mg, 2.5-10 mg, 2.5-7.5 mg, or 2.5-5 mg of letrozole or a pharmaceutical composition thereof.

In some embodiments of the present disclosure, the combined pharmaceutical composition comprises 2.5 mg, 5 mg, 7.5 mg, or 10 mg of letrozole or a pharmaceutical composition thereof. Alternatively, the combined pharmaceutical composition is in the form of a single-administration formulation, and the combined pharmaceutical composition comprises 2.5 mg, 5 mg, 7.5 mg, or 10 mg of letrozole or a pharmaceutical composition thereof.

In some embodiments of the present disclosure, the combined pharmaceutical composition comprises 2.5 mg of letrozole or a pharmaceutical composition thereof. Alternatively, the combined pharmaceutical composition is in the form of a single-administration formulation, and the combined pharmaceutical composition comprises 2.5 mg of letrozole or a pharmaceutical composition thereof.

In some embodiments of the present disclosure, in the combined pharmaceutical composition, the pharmaceutical composition of letrozole is in a single-dose form or in a multi-dose form.

In some embodiments of the present disclosure, in the combined pharmaceutical composition, the pharmaceutical composition of letrozole is in a single-dose form.

In some embodiments of the present disclosure, in the combined pharmaceutical composition, the content of letrozole is a daily dose or a one-day dose.

In some embodiments of the present disclosure, in the combined pharmaceutical composition, the content of letrozole is a once-daily dose or a once-a-day dose.

In some embodiments of the present disclosure, in the combined pharmaceutical composition, the content of letrozole is a once-daily dose or a once-a-day dose, with each dose being a single dose or multiple doses, preferably a single dose.

In some embodiments of the present disclosure, the combined pharmaceutical composition comprises a pharmaceutical composition of letrozole in a single dose of 2.5 mg.

In some embodiments of the present disclosure, the pharmaceutical composition of letrozole comprised in the combined pharmaceutical composition is packaged in a kit, and the kit further comprises an instruction for use of letrozole for treating or preventing breast cancer.

In some embodiments of the present disclosure, the pharmaceutical composition of letrozole comprised in the combined pharmaceutical composition is packaged in a kit, the kit further comprises an instruction for use of letrozole for treating or preventing breast cancer, and the instruction may be the instruction of a commercially available letrozole kit.

In some embodiments of the present disclosure, the combined pharmaceutical composition comprises 20-240 mg of the compound of formula (I) or the pharmaceutically acceptable salt thereof based on the compound of formula (I), and 2.5-12.5 mg of letrozole.

In some embodiments of the present disclosure, the combined pharmaceutical composition comprises 120-180 mg of the compound of formula (I) or the pharmaceutically acceptable salt thereof based on the compound of formula (I), and 2.5-5 mg of letrozole.

In some embodiments of the present disclosure, the combined pharmaceutical composition comprises 20 mg, 40 mg, 60 mg, 80 mg, 100 mg, 120 mg, 150 mg, 180 mg, or 240 mg of a daily dose of the compound of formula (I) or the pharmaceutically acceptable salt thereof based on the compound of formula (I), and comprises 2.5 mg of a daily dose of letrozole.

In some embodiments of the present disclosure, the combined pharmaceutical composition comprises 60 mg, 120 mg, 180 mg, or 240 mg of a daily dose of the compound of formula (I) or the pharmaceutically acceptable salt thereof based on the compound of formula (I), and comprises 2.5 mg of a daily dose of letrozole.

In some embodiments of the present disclosure, the combined pharmaceutical composition comprises a pharmaceutical composition of the compound of formula (I) or the pharmaceutically acceptable salt thereof in a single dose of 50 mg or 60 mg based on the compound of formula (I), and comprises a pharmaceutical composition of letrozole in a single dose of 2.5 mg.

In some embodiments of the present disclosure, the combined pharmaceutical composition comprises a pharmaceutical composition of the compound of formula (I) or the pharmaceutically acceptable salt thereof in a single dose of 60 mg based on the compound of formula (I), and comprises a pharmaceutical composition of letrozole in a single dose of 2.5 mg.

In some embodiments of the present disclosure, the combined pharmaceutical composition comprises the compound of formula (I) or the pharmaceutically acceptable salt thereof or the pharmaceutical composition thereof and letrozole or the pharmaceutical composition thereof in a mass ratio of 1:1 to 100:1, preferably 1.5:1 to 96:1, 5:1 to 90:1, 10:1 to 85:1, 10:1 to 75:1, 20:1 to 75:1, or 24:1 to 72:1, or any ratio within the range described above, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole.

In some embodiments of the present disclosure, the combined pharmaceutical composition is a formulation suitable for administration in a single treatment cycle (e.g., 28 days), and the formulation comprises: 1680-5040 mg (e.g., 3360-5040 mg, preferably 5040 mg) of the compound of formula (I) or the pharmaceutically acceptable salt thereof or a pharmaceutical composition thereof; and 70-140 mg (e.g., 70 mg) of letrozole or a pharmaceutical composition thereof, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole.

In some embodiments of the present disclosure, the combined pharmaceutical composition is a formulation suitable for administration in a single treatment cycle (e.g., 28 days), and the formulation comprises the compound of formula (I) or the pharmaceutically acceptable salt thereof or a pharmaceutical composition thereof and letrozole or a pharmaceutical composition thereof in a mass ratio of 12:1 to 72:1, e.g., 24:1 to 72:1 or 72:1, or any ratio within the range described above, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole.

In some embodiments of the present disclosure, in the combined pharmaceutical composition, the compound of formula (I) or the pharmaceutically acceptable salt thereof and letrozole may be each in the form of a pharmaceutical composition or be in the form of a pharmaceutical composition together.

In another aspect, the present disclosure further provides a kit comprising: (a) a first pharmaceutical composition comprising the compound of formula (I) or the pharmaceutically acceptable salt thereof described herein; and (b) a second pharmaceutical composition comprising letrozole.

In another aspect, the present disclosure further provides a kit of a pharmaceutical composition for use in treating or preventing breast cancer, comprising: (a) a first pharmaceutical composition comprising the compound of formula (I) or the pharmaceutically acceptable salt thereof described herein; and (b) a second pharmaceutical composition comprising letrozole.

In another aspect, the present disclosure further provides a method for treating or preventing breast cancer, comprising administering to an individual in need thereof a therapeutically effective amount of the compound of formula (I) or the pharmaceutically acceptable salt thereof and letrozole, for example, administering to an individual in need thereof a therapeutically effective amount of the combined pharmaceutical composition described above in the present disclosure.

In another aspect, the present disclosure provides a method of administration, comprising: administering to an individual in need thereof a therapeutically effective amount of the compound of formula (I) or the pharmaceutically acceptable salt thereof and letrozole, for example, administering to an individual in need thereof a therapeutically effective amount of the combined pharmaceutical composition described above in the present disclosure, or administering to the individual a therapeutically effective amount of the compound of formula (I) or the pharmaceutically acceptable salt thereof alone and a therapeutically effective amount of letrozole alone.

In another aspect, the present disclosure further provides a combination therapy for use in treating an individual with breast cancer, comprising administering to the individual a therapeutically effective amount of the compound of formula (I) or the pharmaceutically acceptable salt thereof alone and a therapeutically effective amount of letrozole alone.

In another aspect, the present disclosure further provides use of the compound of formula (I) or the pharmaceutically acceptable salt thereof in combination with letrozole for preparing a medicament for treating or preventing breast cancer, for example, use of the combined pharmaceutical composition described above in the present disclosure for preparing a medicament for treating or preventing breast cancer. In some embodiments of the present disclosure, the combined pharmaceutical composition is the combined pharmaceutical composition described above in the present disclosure.

In another aspect, the present disclosure further provides use of the compound of formula (I) or the pharmaceutically acceptable salt thereof in combination with letrozole for treating or preventing breast cancer, for example, use of the combined pharmaceutical composition described above in the present disclosure for treating or preventing breast cancer.

In some embodiments of the present disclosure, in the kit, the method, the combination therapy, or the use, each of the definitions of the compound of formula (I) or the pharmaceutically acceptable salt thereof and letrozole is the same as that in the combined pharmaceutical composition described above, for example, in terms of content, dose, form of presence, form of packaging, or the like.

In some embodiments of the present disclosure, in the method, the combination therapy, or the use, the compound of formula (I) or the pharmaceutically acceptable salt thereof and letrozole are each in the form of a pharmaceutical composition and can be administered simultaneously, separately, concurrently, sequentially, or at intervals.

In some embodiments of the present disclosure, in the method, the combination therapy, or the use, the compound of formula (I) or the pharmaceutically acceptable salt thereof and letrozole each have the same or different treatment cycles. In some specific embodiments of the present disclosure, in the method, the combination therapy, or the use, the compound of formula (I) or the pharmaceutically acceptable salt thereof and letrozole have the same treatment cycle, e.g., a 1-week, 2-week, 3-week or 4-week treatment cycle.

In some embodiments of the present disclosure, in the method, the combination therapy, or the use, the content of the compound of formula (I) or the pharmaceutically acceptable salt thereof in the combined pharmaceutical composition is a daily dose, which is administered by administering the compound of formula (I) or the pharmaceutically acceptable salt thereof once daily.

In some embodiments of the present disclosure, in the method, the combination therapy, or the use, the content of the compound of formula (I) or the pharmaceutically acceptable salt thereof in the combined pharmaceutical composition is a daily dose, wherein the compound of formula (I) or the pharmaceutically acceptable salt thereof is administered in a single dose or in multiple doses, typically in multiple doses; further, the compound of formula (I) or the pharmaceutically acceptable salt thereof is administered once daily.

In some embodiments of the present disclosure, in the method, the combination therapy, or the use, the compound of formula (I) or the pharmaceutically acceptable salt thereof is administered in a daily dose of 60 mg, or in a daily dose of 120 mg, or in a daily dose of 180 mg, or in a daily dose of 240 mg.

In some embodiments of the present disclosure, in the method, the combination therapy, or the use, the compound of formula (I) or the pharmaceutically acceptable salt thereof in the combined pharmaceutical composition is administered in multiple doses consisting of 50 mg or 60 mg single doses of the pharmaceutical composition of the compound of formula (I) or the pharmaceutically acceptable salt thereof, preferably in multiple doses consisting of 60 mg single doses of the pharmaceutical composition of the compound of formula (I) or the pharmaceutically acceptable salt thereof. In some embodiments of the present disclosure, in the method, the combination therapy, or the use, the compound of formula (I) or the pharmaceutically acceptable salt thereof is administered on a regimen of consecutive daily administration.

In some embodiments of the present disclosure, in the method, the combination therapy, or the use, the content of the compound of formula (I) or the pharmaceutically acceptable salt thereof in the combined pharmaceutical composition is a dose per cycle, which is administered by administering the compound of formula (I) or the pharmaceutically acceptable salt thereof daily.

In some embodiments of the present disclosure, in the method, the combination therapy, or the use, the content of letrozole in the combined pharmaceutical composition is a daily dose, which is administered by administering letrozole once daily.

In some embodiments of the present disclosure, in the method, the combination therapy, or the use, the content of letrozole in the combined pharmaceutical composition is a daily dose, wherein letrozole is administered in a single dose or in multiple doses, typically in a single dose; further, letrozole is administered once daily.

In some embodiments of the present disclosure, in the method, the combination therapy, or the use, letrozole is administered in a daily dose of 2.5 mg.

In some embodiments of the present disclosure, in the method, the combination therapy, or the use, letrozole in the combined pharmaceutical composition is administered in a single dose of 2.5 mg of the pharmaceutical composition of letrozole. In some embodiments of the present disclosure, in the method, the combination therapy, or the use, letrozole is administered on a regimen of consecutive daily administration.

In some embodiments of the present disclosure, in the method, the combination therapy, or the use, the content of letrozole in the combined pharmaceutical composition is a dose per cycle, which is administered by administering letrozole daily.

In some embodiments of the present disclosure, in the method, the combination therapy, or the use, 28 days are counted as one treatment cycle, and the compound of formula (I) or the pharmaceutically acceptable salt thereof and letrozole are administered daily on days 1-28 in each treatment cycle.

In some specific embodiments of the present disclosure, in the method, the combination therapy, or the use, 28 days are counted as one treatment cycle, and the compound of formula (I) or the pharmaceutically acceptable salt thereof and letrozole are administered once daily on days 1-28 in each treatment cycle.

In some embodiments of the present disclosure, in the method, the combination therapy, or the use, 28 days are counted as one treatment cycle, the administration is performed once daily for 28 consecutive days, and a total dose of the pharmaceutical composition comprising the compound of formula (I) or the pharmaceutically acceptable salt thereof administered per treatment cycle is 1680-5040 mg. In some embodiments of the present disclosure, the total dose of the pharmaceutical composition comprising the compound of formula (I) or the pharmaceutically acceptable salt thereof is selected from the group consisting of 1680 mg, 3360 mg and 5040 mg or from a range formed by any two of the aforementioned values. In some embodiments of the present disclosure, the total dose of the pharmaceutical composition comprising the compound of formula (I) or the pharmaceutically acceptable salt thereof is preferably 5040 mg.

In some embodiments of the present disclosure, in the method, the combination therapy, or the use, 28 days are counted as one treatment cycle, the administration is performed once daily for 28 consecutive days, and a total dose of the pharmaceutical composition comprising letrozole administered per treatment cycle is 70-210 mg. In some embodiments of the present disclosure, the total dose of the pharmaceutical composition comprising letrozole is selected from the group consisting of 70 mg, 140 mg and 210 mg or from a range formed by any two of the aforementioned values. In some embodiments of the present disclosure, the total dose of the pharmaceutical composition comprising letrozole is preferably 70 mg. In some embodiments of the present disclosure, in the method, the combination therapy, or the use, 28 days are counted as one treatment cycle, the administration is performed once daily for 28 consecutive days, and a total dose of 5040 mg of the pharmaceutical composition comprising the compound of formula (I) or the pharmaceutically acceptable salt thereof and a total dose of 70 mg of the pharmaceutical composition comprising letrozole are administered per treatment cycle.

In embodiments of the present disclosure, the treatment cycle described above is repeated as long as the disease is still under control and the administration regimen is clinically tolerable.

In some embodiments of the present disclosure, letrozole is prepared as a single dose or multiple doses suitable for consecutively administering daily 2.5-7.5 mg or 2.5-5 mg of letrozole to a patient; and the pharmaceutical composition comprising the compound of formula (I) or the pharmaceutically acceptable salt thereof is prepared as a single dose or multiple doses suitable for consecutively administering daily 60 mg, 120 mg, 180 mg, and/or 240 mg based on the compound of formula (I) to a patient.

In some embodiments of the present disclosure, letrozole is prepared as a single dose suitable for consecutively administering daily 2.5 mg of letrozole to a patient; and the pharmaceutical composition comprising the compound of formula (I) or the pharmaceutically acceptable salt thereof is prepared as multiple doses suitable for consecutively administering daily 180 mg based on the compound of formula (I) to a patient.

### Combined pharmaceutical composition of compound of formula (I) or pharmaceutically acceptable salt thereof and anastrozole

In some embodiments of the present disclosure, in the combined pharmaceutical composition, the aromatase inhibitor is anastrozole.

In some embodiments of the present disclosure, the combined pharmaceutical composition comprises 1-5 mg, 1-4 mg, 1-3 mg, or 1-2 mg of anastrozole or a pharmaceutical composition thereof. Alternatively, the combined pharmaceutical composition is in the form of a single-administration formulation, and the combined pharmaceutical composition comprises 1-5 mg, 1-4 mg, 1-3 mg, or 1-2 mg of anastrozole or a pharmaceutical composition thereof.

In some embodiments of the present disclosure, the combined pharmaceutical composition comprises 1 mg, 2 mg, 3 mg, or 4 mg of anastrozole or a pharmaceutical composition thereof. Alternatively, the combined pharmaceutical composition is in the form of a single-administration formulation, and the combined pharmaceutical composition comprises 1 mg, 2 mg, 3 mg, or 4 mg of anastrozole or a pharmaceutical composition thereof.

In some embodiments of the present disclosure, the combined pharmaceutical composition comprises 1 mg of anastrozole or a pharmaceutical composition thereof. Alternatively, the combined pharmaceutical composition is in the form of a single-administration formulation, and the combined pharmaceutical composition comprises 1 mg of anastrozole or a pharmaceutical composition thereof.

In some embodiments of the present disclosure, in the combined pharmaceutical composition, the pharmaceutical composition of anastrozole is in a single-dose form or in a multi-dose form.

In some embodiments of the present disclosure, in the combined pharmaceutical composition, the pharmaceutical composition of anastrozole is in a single-dose form.

In some embodiments of the present disclosure, in the combined pharmaceutical composition, the content of anastrozole is a daily dose or a one-day dose.

In some embodiments of the present disclosure, in the combined pharmaceutical composition, the content of anastrozole is a once-daily dose or a once-a-day dose.

In some embodiments of the present disclosure, in the combined pharmaceutical composition, the content of anastrozole is a once-daily dose or a once-a-day dose, with each dose being a single dose or multiple doses, preferably a single dose.

In some embodiments of the present disclosure, the combined pharmaceutical composition comprises a pharmaceutical composition of anastrozole in a single dose of 1 mg.

In some embodiments of the present disclosure, the pharmaceutical composition of anastrozole comprised in the combined pharmaceutical composition is packaged in a kit, and the kit further comprises an instruction for use of anastrozole for treating or preventing breast cancer.

In some embodiments of the present disclosure, the pharmaceutical composition of anastrozole comprised in the combined pharmaceutical composition is packaged in a kit, the kit further comprises an instruction for use of anastrozole for treating or preventing breast cancer, and the instruction may be the instruction of a commercially available anastrozole kit.

In some embodiments of the present disclosure, the combined pharmaceutical composition comprises 20-240 mg of the compound of formula (I) or the pharmaceutically acceptable salt thereof based on the compound of formula (I), and 1-5 mg of anastrozole.

In some embodiments of the present disclosure, the combined pharmaceutical composition comprises 120-180 mg of the compound of formula (I) or the pharmaceutically acceptable salt thereof based on the compound of formula (I), and 1-2 mg of anastrozole.

In some embodiments of the present disclosure, the combined pharmaceutical composition comprises 20 mg, 40 mg, 60 mg, 80 mg, 100 mg, 120 mg, 150 mg, 180 mg, or 240 mg of a daily dose of the compound of formula (I) or the pharmaceutically acceptable salt thereof based on the compound of formula (I), and comprises 1 mg of a daily dose of anastrozole.

In some embodiments of the present disclosure, the combined pharmaceutical composition comprises 60 mg, 120 mg, 180 mg, or 240 mg of a daily dose of the compound of formula (I) or the pharmaceutically acceptable salt thereof based on the compound of formula (I), and comprises 1 mg of a daily dose of anastrozole.

In some embodiments of the present disclosure, the combined pharmaceutical composition comprises a pharmaceutical composition of the compound of formula (I) or the pharmaceutically acceptable salt thereof in a single dose of 50 mg or 60 mg based on the compound of formula (I), and comprises a pharmaceutical composition of anastrozole in a single dose of 1 mg.

In some embodiments of the present disclosure, the combined pharmaceutical composition comprises a pharmaceutical composition of the compound of formula (I) or the pharmaceutically acceptable salt thereof in a single dose of 60 mg based on the compound of formula (I), and comprises a pharmaceutical composition of anastrozole in a single dose of 1 mg.

In some embodiments of the present disclosure, the combined pharmaceutical composition comprises the compound of formula (I) or the pharmaceutically acceptable salt thereof or the pharmaceutical composition thereof and anastrozole or the pharmaceutical composition thereof in a mass ratio of 4:1 to 240:1, preferably 10:1 to 180:1, 25:1 to 180:1, 30:1 to 180:1, or 60:1 to 180:1, or any ratio within the range described above, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole.

In some embodiments of the present disclosure, the combined pharmaceutical composition is a formulation suitable for administration in a single treatment cycle (e.g., 28 days), and the formulation comprises: 1680-5040 mg (e.g., 3360-5040 mg, preferably 5040 mg) of the compound of formula (I) or the pharmaceutically acceptable salt thereof or a pharmaceutical composition thereof; and 28-140 mg (e.g., 28-56 mg, preferably 28 mg) of anastrozole or a pharmaceutical composition thereof, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole.

In some embodiments of the present disclosure, the combined pharmaceutical composition is a formulation suitable for administration in a single treatment cycle (e.g., 28 days), and the formulation comprises the compound of formula (I) or the pharmaceutically acceptable salt thereof or a pharmaceutical composition thereof and anastrozole or a pharmaceutical composition thereof in a mass ratio of 12:1 to 180:1, e.g., 60:1 to 180:1 or 180:1, or any ratio within the range described above, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole.

In some embodiments of the present disclosure, in the combined pharmaceutical composition, the compound of formula (I) or the pharmaceutically acceptable salt thereof and anastrozole may be each in the form of a pharmaceutical composition or be in the form of a pharmaceutical composition together.

In another aspect, the present disclosure further provides a kit comprising: (a) a first pharmaceutical composition comprising the compound of formula (I) or the pharmaceutically acceptable salt thereof described herein; and (b) a second pharmaceutical composition comprising anastrozole.

In another aspect, the present disclosure further provides a kit of a pharmaceutical composition for use in treating or preventing breast cancer, comprising: (a) a first pharmaceutical composition comprising the compound of formula (I) or the pharmaceutically acceptable salt thereof described herein; and (b) a second pharmaceutical composition comprising anastrozole.

In another aspect, the present disclosure further provides a method for treating or preventing breast cancer, comprising administering to an individual in need thereof a therapeutically effective amount of the compound of formula (I) or the pharmaceutically acceptable salt thereof and anastrozole, for example, administering to an individual in need thereof a therapeutically effective amount of the combined pharmaceutical composition described above in the present disclosure.

In another aspect, the present disclosure further provides a method of administration, comprising administering to an individual in need thereof a therapeutically effective amount of the compound of formula (I) or the pharmaceutically acceptable salt thereof and anastrozole, for example, administering to an individual in need thereof a therapeutically effective amount of the combined pharmaceutical composition described above in the present disclosure, or administering to the individual a therapeutically effective amount of the compound of formula (I) or the pharmaceutically acceptable salt thereof alone and a therapeutically effective amount of anastrozole alone.

In another aspect, the present disclosure further provides a combination therapy for use in treating an individual with breast cancer, comprising administering to the individual a therapeutically effective amount of the compound of formula (I) or the pharmaceutically acceptable salt thereof alone and a therapeutically effective amount of anastrozole alone.

In another aspect, the present disclosure further provides use of the compound of formula (I) or the pharmaceutically acceptable salt thereof in combination with anastrozole for preparing a medicament for treating or preventing breast cancer, for example, use of the combined pharmaceutical composition described above in the present disclosure for preparing a medicament for treating or preventing breast cancer. In some embodiments of the present disclosure, the combined pharmaceutical composition is the combined pharmaceutical composition described above in the present disclosure.

In another aspect, the present disclosure further provides use of the compound of formula (I) or the pharmaceutically acceptable salt thereof in combination with anastrozole for treating or preventing breast cancer, for example, use of the combined pharmaceutical composition described above in the present disclosure for treating or preventing breast cancer.

In some embodiments of the present disclosure, in the kit, the method, the combination therapy, or the use, each of the definitions of the compound of formula (I) or the pharmaceutically acceptable salt thereof and anastrozole is the same as that in the combined pharmaceutical composition described above, for example, in terms of content, dose, form of presence, form of packaging, or the like.

In some embodiments of the present disclosure, in the method, the combination therapy, or the use, the compound of formula (I) or the pharmaceutically acceptable salt thereof and anastrozole are each in the form of a pharmaceutical composition and can be administered simultaneously, separately, concurrently, sequentially, or at intervals.

In some embodiments of the present disclosure, in the method, the combination therapy, or the use, the compound of formula (I) or the pharmaceutically acceptable salt thereof and anastrozole each have the same or different treatment cycles. In some specific embodiments of the present disclosure, in the method, the combination therapy, or the use, the compound of formula (I) or the pharmaceutically acceptable salt thereof and anastrozole have the same treatment cycle, e.g., a 1-week, 2-week, 3-week or 4-week treatment cycle.

In some embodiments of the present disclosure, in the method, the combination therapy, or the use, the content of the compound of formula (I) or the pharmaceutically acceptable salt thereof in the combined pharmaceutical composition is a daily dose, which is administered by administering the compound of formula (I) or the pharmaceutically acceptable salt thereof once daily.

In some embodiments of the present disclosure, in the method, the combination therapy, or the use, the content of the compound of formula (I) or the pharmaceutically acceptable salt thereof in the combined pharmaceutical composition is a daily dose, wherein the compound of formula (I) or the pharmaceutically acceptable salt thereof is administered in a single dose or in multiple doses, typically in multiple doses; further, the compound of formula (I) or the pharmaceutically acceptable salt thereof is administered once daily.

In some embodiments of the present disclosure, in the method, the combination therapy, or the use, the compound of formula (I) or the pharmaceutically acceptable salt thereof is administered in a daily dose of 60 mg, or in a daily dose of 120 mg, or in a daily dose of 180 mg, or in a daily dose of 240 mg.

In some embodiments of the present disclosure, in the method, the combination therapy, or the use, the compound of formula (I) or the pharmaceutically acceptable salt thereof in the combined pharmaceutical composition is administered in multiple doses consisting of 50 mg or 60 mg single doses of the pharmaceutical composition of the compound of formula (I) or the pharmaceutically acceptable salt thereof, preferably in multiple doses consisting of 60 mg single doses of the pharmaceutical composition of the compound of formula (I) or the pharmaceutically acceptable salt thereof. In some embodiments of the present disclosure, in the method, the combination therapy, or the use, the compound of formula (I) or the pharmaceutically acceptable salt thereof is administered on a regimen of consecutive daily administration.

In some embodiments of the present disclosure, in the method, the combination therapy, or the use, the content of the compound of formula (I) or the pharmaceutically acceptable salt thereof in the combined pharmaceutical composition is a dose per cycle, which is administered by administering the compound of formula (I) or the pharmaceutically acceptable salt thereof daily.

In some embodiments of the present disclosure, in the method, the combination therapy, or the use, the content of anastrozole in the combined pharmaceutical composition is a daily dose, which is administered by administering anastrozole once daily.

In some embodiments of the present disclosure, in the method, the combination therapy, or the use, the content of anastrozole in the combined pharmaceutical composition is a daily dose, wherein anastrozole is administered in a single dose or in multiple doses, typically in a single dose; further, anastrozole is administered once daily.

In some embodiments of the present disclosure, in the method, the combination therapy, or the use, anastrozole is administered in a daily dose of 1 mg.

In some embodiments of the present disclosure, in the method, the combination therapy, or the use, anastrozole in the combined pharmaceutical composition is administered in a single dose of 1 mg of the pharmaceutical composition of anastrozole. In some embodiments of the present disclosure, in the method, the combination therapy, or the use, anastrozole is administered on a regimen of consecutive daily administration.

In some embodiments of the present disclosure, in the method, the combination therapy, or the use, the content of anastrozole in the combined pharmaceutical composition is a dose per cycle, which is administered by administering anastrozole daily.

In some embodiments of the present disclosure, in the method, the combination therapy, or the use, 28 days are counted as one treatment cycle, and the compound of formula (I) or the pharmaceutically acceptable salt thereof and anastrozole are administered daily on days 1-28 in each treatment cycle.

In some specific embodiments of the present disclosure, in the method, the combination therapy, or the use, 28 days are counted as one treatment cycle, and the compound of formula (I) or the pharmaceutically acceptable salt thereof and anastrozole are administered once daily on days 1-28 in each treatment cycle.

In some embodiments of the present disclosure, in the method, the combination therapy, or the use, 28 days are counted as one treatment cycle, the administration is performed once daily for 28 consecutive days, and a total dose of the pharmaceutical composition comprising the compound of formula (I) or the pharmaceutically acceptable salt thereof administered per treatment cycle is 1680-5040 mg. In some embodiments, the total dose of the pharmaceutical composition comprising the compound of formula (I) or the pharmaceutically acceptable salt thereof is selected from the group consisting of 1680 mg, 3360 mg and 5040 mg or from a range formed by any two of the aforementioned values. In some embodiments, the total dose of the pharmaceutical composition comprising the compound of formula (I) or the pharmaceutically acceptable salt thereof is preferably 5040 mg.

In some embodiments of the present disclosure, in the method, the combination therapy, or the use, 28 days are counted as one treatment cycle, the administration is performed once daily for 28 consecutive days, and a total dose of the pharmaceutical composition comprising anastrozole administered per treatment cycle is 28-140 mg. In some embodiments of the present disclosure, the total dose of the pharmaceutical composition comprising anastrozole is selected from the group consisting of 28 mg, 56 mg and 84 mg or from a range formed by any two of the aforementioned values. In some embodiments of the present disclosure, the total dose of the pharmaceutical composition comprising anastrozole is preferably 28 mg. In some embodiments of the present disclosure, in the method, the combination therapy, or the use, 28 days are counted as one treatment cycle, the administration is performed once daily for 28 consecutive days, and a total dose of 5040 mg of the pharmaceutical composition comprising the compound of formula (I) or the pharmaceutically acceptable salt thereof and a total dose of 28 mg of the pharmaceutical composition comprising anastrozole are administered per treatment cycle.

In embodiments of the present disclosure, the treatment cycle described above is repeated as long as the disease is still under control and the administration regimen is clinically tolerable.

In some embodiments of the present disclosure, anastrozole is prepared as a single dose or multiple doses suitable for consecutively administering daily 1-3 mg or 1-2 mg of anastrozole to a patient; and the pharmaceutical composition comprising the compound of formula (I) or the pharmaceutically acceptable salt thereof is prepared as a single dose or multiple doses suitable for consecutively administering daily 60 mg, 120 mg, 180 mg, and/or 240 mg based on the compound of formula (I) to a patient.

In some embodiments of the present disclosure, anastrozole is prepared as a single dose suitable for consecutively administering daily 1 mg of anastrozole to a patient; and the pharmaceutical composition comprising the compound of formula (I) or the pharmaceutically acceptable salt thereof is prepared as multiple doses suitable for consecutively administering daily 180 mg based on the compound of formula (I) to a patient.

In some embodiments of the present disclosure, the breast cancer is selected from HR-positive breast cancer.

In some embodiments of the present disclosure, the breast cancer is selected from HER2-negative breast cancer. In some embodiments of the present disclosure, the breast cancer is selected from the group consisting of HR-positive and HER2-negative breast cancer.

In some embodiments of the present disclosure, the breast cancer is selected from the group consisting of locally advanced and/or metastatic breast cancer. In some embodiments of the present disclosure, the breast cancer is selected from the group consisting of HER2-negative locally advanced and/or metastatic breast cancer.

In some embodiments of the present disclosure, the breast cancer is selected from the group consisting of HR-positive and HER2-negative locally advanced and/or metastatic breast cancer.

In some embodiments of the present disclosure, the breast cancer is selected from the group consisting of HR-positive and HER2-negative locally advanced and/or metastatic breast cancer unable to receive radical surgery or radiotherapy. In some embodiments of the present disclosure, the patient with breast cancer is a patient with HR-positive and HER2-negative locally advanced and/or metastatic breast cancer unable to receive radical surgery or radiotherapy.

In some embodiments of the present disclosure, the breast cancer is selected from the group consisting of postmenopausal or premenopausal/perimenopausal breast cancer. In some embodiments of the present disclosure, the patient with breast cancer is a patient with postmenopausal or premenopausal/perimenopausal breast cancer. In some embodiments of the present disclosure, the breast cancer is selected from the group consisting of postmenopausal or premenopausal/perimenopausal HR-positive and HER2-negative locally advanced and/or metastatic breast cancer. In some embodiments of the present disclosure, the patient with breast cancer is a patient with postmenopausal or premenopausal/perimenopausal HR-positive and HER2-negative locally advanced and/or metastatic breast cancer.

In some embodiments of the present disclosure, the breast cancer is selected from the group consisting of breast cancer previously received bilateral ovariectomy. In some embodiments of the present disclosure, the patient with breast cancer is a patient with breast cancer previously received bilateral ovariectomy.

In some embodiments of the present disclosure, the breast cancer is selected from the group consisting of postmenopausal or premenopausal/perimenopausal breast cancer previously received bilateral ovariectomy. In some embodiments of the present disclosure, the patient with breast cancer is a patient with postmenopausal or premenopausal/perimenopausal breast cancer previously received bilateral ovariectomy.

In some embodiments of the present disclosure, the breast cancer is selected from the group consisting of postmenopausal or premenopausal/perimenopausal HR-positive and HER2-negative locally advanced and/or metastatic breast cancer previously received bilateral ovariectomy. In some embodiments of the present disclosure, the patient with breast cancer is a patient with postmenopausal or premenopausal/perimenopausal HR-positive and HER2-negative locally advanced and/or metastatic breast cancer previously received bilateral ovariectomy.

In some embodiments of the present disclosure, the breast cancer is selected from the group consisting of postmenopausal or premenopausal/perimenopausal HR-positive and HER2-negative locally advanced and/or metastatic breast cancer unable to receive radical surgery or radiotherapy. In some embodiments of the present disclosure, the patient with breast cancer is a patient with postmenopausal or premenopausal/perimenopausal HR-positive and HER2-negative locally advanced and/or metastatic breast cancer unable to receive radical surgery or radiotherapy.

In some embodiments of the present disclosure, the HR-positive includes estrogen receptor (ER)-positive and/or progesterone receptor (PR)-positive, and is defined as follows: the proportion of positive staining tumor cells in all tumor cells is more than or equal to 1%.

In some embodiments of the present disclosure, the HER2-negative is defined as follows: HER2 is 0/1+ as detected by immunohistochemical staining (IHC); if HER2 is 2+ as detected, fluorescence in situ hybridization (FISH) should be performed to confirm that the result is negative, or only FISH assay is performed and the result is negative.

In some embodiments of the present disclosure, the breast cancer is selected from the group consisting of HR-positive and HER2-negative locally advanced and/or metastatic breast cancer previously received chemotherapy no more than first-line.

In some embodiments of the present disclosure, the breast cancer is selected from the group consisting of HR-positive and HER2-negative breast cancer that relapse or progress during an adjuvant endocrine therapy or within 1 year after completion of an adjuvant endocrine therapy and have not received endocrine therapy subsequently.

In some embodiments of the present disclosure, the breast cancer is selected from the group consisting of HR-positive and HER2-negative breast cancer that relapse or progress over 1 year after completion of an adjuvant endocrine therapy and subsequently progress again after receiving an advanced endocrine therapy; an aromatase inhibitor cannot be used in the advanced endocrine therapy.

In some embodiments of the present disclosure, the breast cancer is selected from the group consisting of breast cancer with disease progression after receiving an advanced endocrine therapy for a primary or metastatic disease; an aromatase inhibitor cannot be used in the advanced endocrine therapy.

In some embodiments of the present disclosure, the breast cancer is selected from the group consisting of HR-positive and HER2-negative locally advanced and/or metastatic breast cancer that has not previously received any whole-body systemic anti-tumor therapy for local lesion recurrence or metastatic diseases.

In some embodiments of the present disclosure, completion of the adjuvant endocrine therapy refers to consecutive treatment for at least 2 years and then interruption.

In some embodiments of the present disclosure, the endocrine therapy refers to a therapy with tamoxifen, toremifene, fulvestrant, goserelin, and leuprorelin.

In some embodiments of the present disclosure, the endocrine therapy refers to a therapy with tamoxifen and goserelin. The active ingredients in the pharmaceutical combination disclosed herein can be formulated independently, or some or all of the active ingredients are co-formulated with a pharmaceutically acceptable carrier and/or excipient. The pharmaceutical combination disclosed herein may further comprise an additional therapeutic agent. In some embodiments of the present disclosure, the additional therapeutic agent may be a therapeutic agent known in the art for cancer, preferably a therapeutic agent for breast cancer.

In some embodiments of the present disclosure, the cycle is 28 days.

The amount of the compound of formula (I) or the pharmaceutically acceptable salt thereof administered can be determined according to the severity of the disease, the response of the disease, any treatment-related toxicity, and the age and health of a patient.

The compound of formula (I) or the pharmaceutically acceptable salt thereof can be administered via multiple routes of administration including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular, intraperitoneal, and intrathecal administrations. In a specific embodiment, the compound of formula (I) or the pharmaceutically acceptable salt thereof is administered orally. In some embodiments of the present disclosure, the compound of formula (I) or the pharmaceutically acceptable salt thereof is administered on a regimen of consecutive daily oral administration.

The compound of formula (I) or the pharmaceutically acceptable salt thereof can be administered once or multiple times daily. In some embodiments of the present disclosure, the compound of formula (I) or the pharmaceutically acceptable salt thereof is administered once daily. The compound of formula (I) or the pharmaceutically acceptable salt thereof may also be administered in a single-dose form or in a multi-dose form. In one embodiment, the compound of formula (I) or the pharmaceutically acceptable salt thereof is administered in multiple doses once daily.

In some embodiments of the present disclosure, the compound of formula (I) or the pharmaceutically acceptable salt thereof is administered in the form of a multi-dose oral solid formulation once daily. In one embodiment, the compound of formula (I) or the pharmaceutically acceptable salt thereof is administered in multiple doses once daily.

The dosage regimen can be determined comprehensively depending on the activity and toxicity of the medicament, tolerance of a patient, etc.

### Compound of formula (I) or pharmaceutically acceptable salt thereof

As used in the present disclosure, the pharmaceutically acceptable salt of the compound of formula (I) may be a maleate of the compound of formula (I) (such as a monomaleate of the compound of formula (I)).

The dosage of the compound of formula (I) or the pharmaceutically acceptable salt thereof referred to in the present disclosure is based on the molecular weight of the compound of formula (I), unless otherwise stated.

The compound of formula (I) or the pharmaceutically acceptable salt thereof used in the present disclosure may be prepared by methods known in the art, for example, by reference to WO2016141881.

### Pharmaceutical composition of compound of formula (I) or pharmaceutically acceptable salt thereof

In some embodiments of the present disclosure, a single dose of the pharmaceutical composition of the compound of formula (I) or the pharmaceutically acceptable salt thereof may be 50 mg or 60 mg, based on the compound of formula (I).

In some embodiments of the present disclosure, a single dose of the pharmaceutical composition of the compound of formula (I) or the pharmaceutically acceptable salt thereof is 60 mg.

The dosage regimen can be determined comprehensively depending on the activity and toxicity of the medicament, tolerance of a patient, etc.

In some embodiments of the present disclosure, the pharmaceutical composition comprising the compound of formula (I) or the pharmaceutically acceptable salt thereof further comprises a pharmaceutically acceptable excipient. The pharmaceutically acceptable excipient includes a filler, an absorbent, a wetting agent, a binder, a disintegrant, a lubricant, and the like. In some embodiments of the present disclosure, the pharmaceutical composition includes, but is not limited to, a formulation suitable for oral, parenteral and topical administration. In some embodiments, the pharmaceutical composition is a formulation suitable for oral administration. In some embodiments, the pharmaceutical composition is a solid formulation suitable for oral administration. In some embodiments, the pharmaceutical composition includes, but is not limited to, a tablet and a capsule.

In some embodiments of the present disclosure, the pharmaceutical composition is a solid pharmaceutical combination.

In some embodiments of the present disclosure, the pharmaceutical composition is a capsule.

In some embodiments of the present disclosure, the pharmaceutical composition of the compound of formula (I) or the pharmaceutically acceptable salt thereof is a solid pharmaceutical composition of the compound of formula (I).

In some embodiments of the present disclosure, the pharmaceutical composition of the compound of formula (I) or the pharmaceutically acceptable salt thereof is a capsule of the compound of formula (I).

The pharmaceutical composition of the compound of formula (I) or the pharmaceutically acceptable salt thereof disclosed herein can be manufactured using methods well known in the art, such as conventional mixing, dissolving, granulating, dragee-making, levigating, emulsifying, and lyophilizing.

A solid oral composition can be prepared by conventional mixing, filling or tableting. For example, it can be obtained by the following method: mixing the active compounds with a solid excipient, optionally grinding the resulting mixture, adding an additional suitable excipient if desired, and processing the mixture into granules to give the core parts of tablets or dragees or to perform capsule filling to give capsules. Suitable excipients include, but are not limited to: binders, diluents, disintegrants, lubricants, glidants, sweeteners or flavoring agents, and the like.

### Letrozole

As used in the present disclosure, letrozole is known by the chemical name of 1-[bis(4-cyanophenyl)methyl]-1,2,4-triazole, which has the following structural formula:

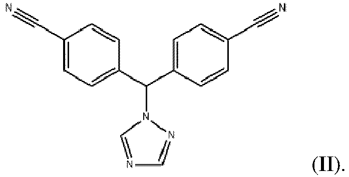

### Pharmaceutical composition of letrozole

In some embodiments of the present disclosure, the pharmaceutical composition of letrozole further comprises a pharmaceutically acceptable excipient. In some embodiments, the pharmaceutically acceptable excipient includes a filler, an absorbent, a wetting agent, a binder, a diluent, a disintegrant, a lubricant, a glidant, a sweetener, a flavoring agent, or the like.

In some embodiments of the present disclosure, the pharmaceutical composition is a solid pharmaceutical composition.

In some embodiments of the present disclosure, the pharmaceutical composition is a tablet.

In some embodiments of the present disclosure, the pharmaceutical composition is an oral tablet.

In some embodiments of the present disclosure, a single dose of the pharmaceutical composition of letrozole is 2.5 mg.

### Anastrozole

As used in the present disclosure, anastrozole is known by the chemical name of a,a,a',a'-tetramethyl-5-(1H-1,2,4-triazol-1-methyl)-1,3-benzenediacetonitrile, which has the following structural formula:

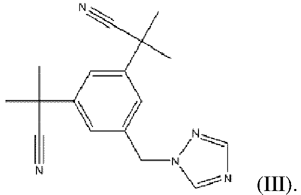

### Pharmaceutical composition of anastrozole

In some embodiments of the present disclosure, the pharmaceutical composition of anastrozole further comprises a pharmaceutically acceptable excipient. In some embodiments, the pharmaceutically acceptable excipient includes a filler, an absorbent, a wetting agent, a binder, a diluent, a disintegrant, a lubricant, a glidant, a sweetener, a flavoring agent, or the like.

In some embodiments of the present disclosure, the pharmaceutical composition is a solid pharmaceutical composition.

In some embodiments of the present disclosure, the pharmaceutical composition is a tablet.

In some embodiments of the present disclosure, the pharmaceutical composition is an oral tablet.

In some embodiments of the present disclosure, a single dose of the pharmaceutical composition of anastrozole is 1 mg.

### Administration

The content below is not intended to limit the administration of the combined pharmaceutical composition disclosed herein.

The active ingredients in the combined pharmaceutical composition disclosed herein can be administered independently, or some or all of the active ingredients are co-administered in various proper routes, including, but not limited to, oral administration or parenteral administration (by intravenous, intramuscular, topical, or subcutaneous routes). In some embodiments of the present disclosure, the active ingredients in the combined pharmaceutical composition disclosed herein can be administered independently, or some or all of the active ingredients are co-administered orally.

The active ingredients in the combined pharmaceutical composition disclosed herein can be formulated independently in suitable dosage forms, or some or all of the components are co-formulated in a suitable dosage form including, but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant, and dosage forms of sustained-released preparations for oral or non-oral administration.

Any combination or substitution may be made between the embodiments described above herein and the various features included therein, and the resulting embodiments also fall within the protection scope of the present application.

### Technical effects

Generally, use of the combined pharmaceutical composition disclosed herein described above will provide:
(1) better efficacy in controlling tumor growth or even eliminating tumors as compared with either drug of the combination administered alone;
(2) fewer doses as compared with either drug of the combination administered alone;
(3) good tolerability in patients, and fewer adverse effects and/or complications as compared with either drug administered alone;
(4) a higher disease control rate in patients treated;
(5) longer survivals (e.g., median survival time, progression-free survival, or overall survival) in patients treated;
(6) longer survivals (e.g., median survival time, progression-free survival, or overall survival) in patients treated as compared with standard chemotherapies;
(7) a longer duration of response (DOR); and/or
(8) better activity in treating tumors or proliferative diseases and better anti-tumor synergistic effect, as compared with either drug of the combination administered alone.

The "clinical benefits" of the combined pharmaceutical composition disclosed herein include, but are not limited to: prolonged progression-free survival (PFS), prolonged overall survival (OS), improved objective response rate (ORR), improved disease control rate (DCR), reduced number and/or degree of adverse effects, decreased distant metastasis rate, decreased local control rate, and the like for clinical patients.

### Definitions and explanations

As used herein, the term "combined pharmaceutical composition" refers to a combination of two or more active ingredients (administered as the respective active ingredients themselves, or as their respective derivatives like pharmaceutically acceptable salts or esters, prodrugs, or compositions) that are administered simultaneously or sequentially. As used herein, the terms "combined pharmaceutical composition" and "pharmaceutical combination" are used interchangeably.

The word "comprise", and variants thereof such as "comprises" or "comprising", and equivalents thereof shall be understood in an open, non-exclusive sense, i.e., "includes but is not limited to", indicating that in addition to the listed elements, components and procedures, other unspecified elements, components and procedures may also be encompassed.

The term "patient" or "individual/subject" refers to a mammal, e.g., a primate (human, macaque, chimpanzee, etc.), rodent (mouse, rat, rabbit, etc.), feline, canine, etc., preferably a human. In some embodiments of the present application, the patient and the individual are patients who have failed or lack a standard treatment.

The term "pharmaceutically acceptable" refers to a carrier or an excipient that is used in the preparation of a pharmaceutical composition. The carrier or the excipient is generally safe, non-toxic, and desirable biologically and otherwise, and inclusion of the substance is acceptable for pharmaceutical use in humans.

The term "therapeutically effective amount" means an amount of a compound that, when administered to a human for treating a disease, is sufficient to treat the disease.

The term "treat", "treating", or "treatment" means administering the compound or formulation of the present application to ameliorate, alleviate, or eliminate a disease or one or more symptoms associated with the disease, and includes: (i) inhibiting the disease or disease state, i.e., arresting or delaying its development; and (ii) relieving the disease or disease state, i.e., causing regression of the disease or disease state.

The term "prevent", "preventing", or "prevention" means administering the compound or formulation of the present application to prevent a disease or one or more symptoms associated with the disease, and includes: preventing the occurrence of the disease or disease state in a mammal, particularly when such a mammal is predisposed to the disease state but has not yet been diagnosed with it.

The term "whole-body therapy" refers to a therapy in which a drug substance is transported through the bloodstream to reach and affect cells throughout the body.

The term "systemic therapy" refers to systemic chemotherapy, and whole-body or local radiotherapy.

The term "first-line therapy" refers to a therapy with drugs that are the first or standard choice according to a patient's conditions. As used herein, an "adverse event" (AE) is any adverse and often unintended or undesirable sign (including abnormal laboratory findings), symptom, or disease associated with the use of medical therapy. For example, an adverse event may be associated with activation of the immune system or expansion of immune system cells (e.g., T cells) in response to treatment. The medical treatment may have one or more related AEs, and each AE may have the same or a different severity level. Reference to a method capable of "altering an adverse event" refers to a treatment regimen that reduces the incidence and/or severity of one or more AEs associated with the use of a different treatment regimen.

The use of alternatives (e.g., "or") shall be understood to refer to any one, two, or any combination of the alternatives. As used herein, the indefinite article "a" or "an" shall be understood to mean "one or more" of any listed or enumerated components.

The term "pharmaceutically acceptable excipient" refers to those excipients that do not have a significant irritating effect on an organic entity and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The terms "administer", "administration" and "administering" refer to physically introducing the composition comprising a therapeutic agent to an individual using any of a variety of methods and delivery systems known to those skilled in the art. In certain embodiments, the administration is oral administration.

The term "daily dose" refers to a dose administered to a patient per day.

The term "one-day dose" refers to a dose administered to a patient on one day.

The term "single dose" or "unit formulation" refers to the smallest unit of packaging of a pharmaceutical product comprising a certain amount of active ingredients; for example, in a box of seven capsules, each capsule is a single dose or a unit formulation; in a box of seven tablets, each tablet is a single dose or a unit formulation.

The term "multiple doses" consists of multiple single doses. As used herein, "combined use" or "use in combination" means that two or more active substances may be administered to an individual simultaneously, concurrently, or sequentially in any order as a single formulation.

The term "pharmaceutical composition" refers to a mixture consisting of one or more of the active ingredients or pharmaceutical combinations thereof of the present application and a pharmaceutically acceptable excipient. The pharmaceutical composition is intended to facilitate the administration of the compound or the pharmaceutical combination thereof of the present application to an individual.

The terms "day", "daily", etc., when referred to in an administration regimen, refer to the time within a calendar day that starts at midnight and ends at the next midnight.

The term "recurrent" cancer is one that regenerates at an initial site or a distant site after being responsive to initial treatment (e.g., surgery). A "locally recurrent" cancer is one that occurs, after treatment, at the same location as the previously treated cancer.

The term "unresectable" cancer is one that cannot be removed by surgery.

The term "metastatic" cancer refers to one that spreads from one part of the body (e.g., the lung) to another part of the body.

As used herein, "combined use" or "use in combination" means that two or more active substances may be administered to a subject as a mixture, simultaneously as a single formulation, or sequentially in any order as a single formulation.

Unless otherwise specified clearly herein, singular terms encompass plural terms, and vice versa. Similarly, unless otherwise specified clearly herein, the word "or" is intended to include "and", and vice versa.

Unless otherwise stated herein, parameter values representing amounts of ingredients or physicochemical properties or reaction conditions and the like are to be understood as being modified in all cases by the term "about". When the term "about" is used to describe the present application, the term "about" indicates that there is an error value; for example, it means varying within a range of ±5%, such as ±1% or ±0.1%, of a particular value. All patents, patent applications and other identified publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

### Example

The following specific examples are intended to allow those skilled in the art to clearly understand and implement the present disclosure. These specific examples should not be considered as limitations on the scope of the present disclosure, but merely as exemplary descriptions and representatives of the present disclosure.

### Experimental Example 1. Clinical Trial

The study involved 2 cohorts, namely cohort I and cohort II, and the study drugs were the compound of formula (I) and an aromatase inhibitor in combination for both cohorts. Subjects with HR-positive and HER2-negative locally advanced and/or metastatic breast cancer were enrolled, with 30-60 subjects enrolled in each cohort. The preliminary efficacy and safety of the compound of formula (I) in combination with the aromatase inhibitor were evaluated. The aromatase inhibitor is selected from the group consisting of letrozole and anastrozole, and the two drugs are each administered to 15-30 subjects per cohort.

### 1.1. Inclusion criteria:

1) Subjects with voluntary participation, signed informed consent, and good compliance;
2) Aged 18-75 years (when signing the informed consent); ECOG PS score: 0-1; expected survival time of more than 3 months;
3) Postmenopausal or premenopausal/perimenopausal female patients;
4) Patients with breast cancer diagnosed as HR-positive and HER2-negative by pathological examination;
5) Subjects enrolled in cohort I relapsed or progressed during an adjuvant endocrine therapy or after completion of an adjuvant endocrine therapy;
6) Completion of an adjuvant endocrine therapy in cohort I was defined as consecutive treatment for at least 2 years and then interruption;
7) Subjects enrolled in cohort II had not previously received any whole-body systemic anti-tumor therapy for local lesion recurrence or metastatic diseases;
8) Having at least one measurable lesion as confirmed according to RECIST 1.1 criteria;
9) With good main organ functions meeting the following criteria:
   Blood routine examination criteria (no blood transfusion and no correction using hematopoietic stimulating drugs within the last 7 days before screening):
      a) Hemoglobin (HB) ≥ 100 g/L;
      b) Absolute value of neutrophil count (NEUT) ≥ 1.5 × 10⁹/L;
      c) Platelet count (PLT) ≥ 90 × 10⁹/L.
   Biochemical test results should meet the following criteria:
      a) Total bilirubin (TBIL) ≤ 2.5 times of upper limit of normal (ULN);
      b) Alanine aminotransferase (ALT) and aspartate aminotransferase (AST) ≤ 2.5 × ULN. If liver metastasis is accompanied, ALT and AST ≤ 5 × ULN;
      c) Serum creatinine (CR) ≤ 1.5 × ULN or creatinine clearance rate (CCR) ≥ 60 mL/min.
   Blood coagulation function examination should meet the following criteria:
      Prothrombin time (PT), activated partial thromboplastin time (APTT), and international normalized ratio (INR) ≤ 1.5 × ULN (not previously received anticoagulant therapy);
      Heart color ultrasound evaluation: left ventricular ejection fraction (LVEF) ≥ 50%;
10) Female subjects of child-bearing age should agree to take contraceptive measures (such as intrauterine devices, contraceptives, or condoms) during the study and within 6 months after the study; serum pregnancy test results should be negative within 7 days before the study enrollment, and the subjects must not be breastfeeding.

### 1.2. Test drug

Capsules of the compound of formula (I): strength: 60 mg, provided by Chia Tai Tianqing Pharmaceutical Group Co., Ltd.

Letrozole tablets: strength: 2.5 mg, Yimeishu^{®} purchased from Hisun Pharmaceutical Co., Ltd.

Anastrozole tablets: strength: 1 mg, Yishuzhi^{®} purchased from Hisun Pharmaceutical Co., Ltd.

### 1.3. Administration regimen

(1) Compound of formula (I):
   Capsules of the compound of formula (I): 180 mg (based on the mass of compound of formula (I)), oral administration at fasting, once daily, with 28 consecutive days of administration counted as one treatment cycle.
(2) Aromatase inhibitor: letrozole or anastrozole may be selected for administration on the specific regimen as follows:
   Letrozole tablets: 2.5 mg (based on the mass of letrozole), oral administration, once daily, with 28 consecutive days of administration counted as one treatment cycle. The letrozole tablets were administered together with the capsules of the compound of formula (I).
   Anastrozole tablets: 1 mg (based on the mass of anastrozole), oral administration, once daily, with 28 consecutive days of administration counted as one treatment cycle. The anastrozole tablets were administered together with the capsules of the compound of formula (I).

### 1.4. Evaluation criteria

Effectiveness evaluation criteria: disease status was determined using the RECIST 1.1 criteria.

Safety evaluation criteria: the severity of adverse events was determined using the NCI-CTC AE 5.0 criteria.

### 1.5. Results of trial

### 1.5.1. Safety

A statistical analysis of the safety in 43 subjects showed that the main adverse events were gastrointestinal reactions, and most of the TRAEs were grade 1-2, which could be controlled after symptomatic treatment; the overall incidence of grade 3 TRAEs was only 27.9%; and no grade 4-5 TRAEs and treatment-related deaths were observed.

### 1.5.2. Efficacy

Of the 43 subjects, 38 subjects were enrolled in cohort II and the others were enrolled in cohort I. 35 subjects (cohort II: 32 subjects, cohort I: 3 subjects) were available for efficacy evaluation. Efficacy results showed that the ORR was 65.7% (23/35), wherein the subjects in cohort II had an effective rate of 65.6% (21/32), which was consistent with the ORR of overall population, and the clinical benefit rate (CBR) of cohort II was 90.6% (29/32); the subjects in cohort I had an effective rate of 66.7% (2/3), which was consistent with the ORR of overall population, and the clinical benefit rate (CBR) of cohort I was 100% (3/3).

Conclusion: the combined pharmaceutical composition of the present disclosure can exhibit good clinical benefit. The following is an overview of representative subjects:

| Subject No. | Dosage regimen | Cohort | Administration cycle | Efficacy evaluation | | | | | |
|---|---|---|---|---|---|---|---|---|---|
| | | | | C2 | C4 | C6 | C8 | C10 | C12 |
| 1 | Capsules of the compound of formula (I): 180 mg/dose, once daily, with 28 days counted as one treatment cycle; | 2 | C13 | SD (-21.4%*) | PR (-41.6%*) | NA | PR (-48.9%*) | PR (-43.3%*) | PR (-53.7%*) |
| | letrozole: 2.5 mg/dose, once daily, with 28 days counted as one treatment cycle. | | | | | | | | |
| 2 | Same as No. 1 | 2 | C13 | SD (-20% *) | SD (-25%*) | NA | PR (-40%*) | PR (-40%*) | PR (-40%*) |
| 3 | Same as No. 1 | 2 | C13 | PR (-51.9%*) | PR (-51.9%*) | PR (-51.9%*) | PR (-51.9%*) | PR (-51.9%*) | PR (-51.9%*) |
| 4 | Same as No. 1 | 2 | C11 | PR (-63.6%*) | PR (-63.6%*) | PR (-63.6%*) | PR (-63.6%*) | PR (-63.6%*) | -- |
| 5 | Same as No. 1 | 2 | C9 | PR (-31.5%*) | PR (-44.7%*) | PR (-58.8%*) | PR (-62.6%*) | -- | -- |
| 6 | Capsules of the compound of formula (I): 180 mg/dose, once daily, with 28 days counted as one treatment cycle; | 2 | C12 | PR (-31.4%*) | PR (-57.6%*) | NA | PR (-64.9%*) | PR (-64.9%*) | -- |
| | anastrozole: 1 mg/dose, once daily, with 28 days counted as one treatment cycle. | | | | | | | | |
| 7 | Same as No. 6 | 2 | C12 | PR (-54.1 % *) | PR (-39.5%*) | PR (-39.5%*) | PR (-39.5%*) | PR (-53.4%*) | - |
| 8 | Same as No. 6 | 2 | C11 | SD (-12.1 %*) | PR (-33.5%*) | PR (-40.6%*) | PR (-60.8%*) | PR (-58.1*) | - |
| 9 | Same as No. 6 | 2 | C9 | PR (-54.1 % *) | PR (-80.7%*) | PR (-73.3%*) | PR (-71.6%*) | -- | -- |
| 10 | Same as No. 6 | 2 | C9 | SD (-27.0%*) | PR (-34.2%*) | PR (-37.8%*) | PR (-50.3%*) | -- | -- |
| 11 | Capsules of the compound of formula (I): 180 mg/dose, once daily, with 28 days counted as one treatment cycle; | 1 | C12 | SD (-27.3%*) | PR (-63.6%*) | PR (-63.6%*) | PR (-63.6%*) | PR(-63.6%*) | -- |
| | letrozole: 2.5 mg/dose, once daily, with 28 days counted as one treatment cycle. | | | | | | | | |
| 12 | Same as No. 11 | 1 | C11 | SD (-29.4%*) | SD (-29.4%*) | PR (-47%*) | NA | NA | - |

| | | | | | | | | | |
|---|---|---|---|---|---|---|---|---|---|
| Note: "NA" means not evaluated; "*" indicates tumor volume reduction percentage; and "--" indicates that the tumor evaluation point has not been reached. | | | | | | | | | |

Those skilled in the art will recognize that the scope of the present application is not limited to the various embodiments and examples described above. Instead, various modifications, substitutions, or recombinations can be made without departing from the spirit and concept of the present application, all of which fall within the protection scope of the present application.

## Claims

1. A combined pharmaceutical composition, comprising a compound of formula (I) or a pharmaceutically acceptable salt thereof and an aromatase inhibitor:

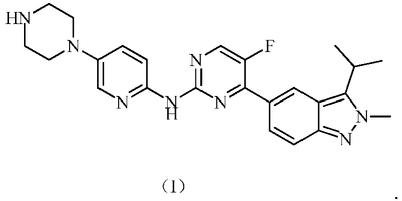

2. The combined pharmaceutical composition according to claim 1, wherein the compound of formula (I) or the pharmaceutically acceptable salt thereof and the aromatase inhibitor are packaged in a kit, and the kit further comprises an instruction for use of the compound of formula (I) or the pharmaceutically acceptable salt thereof in combination with the aromatase inhibitor for treating or preventing breast cancer.

3. The combined pharmaceutical composition according to claim 1, comprising a pharmaceutical composition of the compound of formula (I) or the pharmaceutically acceptable salt thereof, and a pharmaceutical composition of the aromatase inhibitor.

4. The combined pharmaceutical composition according to any one of claims 1 to 3, wherein the aromatase inhibitor is selected from the group consisting of letrozole and anastrozole.

5. The combined pharmaceutical composition according to any one of claims 1 to 4, comprising 20-240 mg, 40-180 mg, 60-180 mg, 80-180 mg, 100-180 mg, 120-180 mg, or 150-180 mg of the compound of formula (I) or the pharmaceutically acceptable salt thereof based on the compound of formula (I), or a pharmaceutical composition thereof; or comprising 20 mg, 40 mg, 60 mg, 80 mg, 100 mg, 120 mg, 150 mg, and/or 240 mg of the compound of formula (I) or the pharmaceutically acceptable salt thereof based on the compound of formula (I), or a pharmaceutical composition thereof.

6. The combined pharmaceutical composition according to claim 4 or 5, wherein the pharmaceutical composition of the compound of formula (I) is in a single dose or in multiple doses, preferably in multiple doses; and/or
the pharmaceutical composition of letrozole or the pharmaceutical composition of anastrozole is in a single dose.

7. The combined pharmaceutical composition according to claim 4, comprising 2.5-12.5 mg, 2.5-10 mg, 2.5-7.5 mg, or 2.5-5 mg of letrozole or a pharmaceutical composition thereof; or comprising 2.5 mg, 5 mg, 7.5 mg, or 10 mg of letrozole or a pharmaceutical composition thereof; or comprising 1-5 mg, 1-4 mg, 1-3 mg, or 1-2 mg of anastrozole or a pharmaceutical composition thereof; or comprising 1 mg, 2 mg, 3 mg, or 4 mg of anastrozole or a pharmaceutical composition thereof.

8. The combined pharmaceutical composition according to claim 7, wherein the combined pharmaceutical composition comprises the compound of formula (I) or the pharmaceutically acceptable salt thereof or the pharmaceutical composition thereof and letrozole or the pharmaceutical composition thereof in a mass ratio of 1:1 to 100:1, preferably 1.5:1 to 96:1, 5:1 to 90:1, 10:1 to 85:1, 10:1 to 75:1, 20:1 to 75:1, or 24:1 to 72:1, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole; or
the combined pharmaceutical composition comprises the compound of formula (I) or the pharmaceutically acceptable salt thereof or the pharmaceutical composition thereof and anastrozole or the pharmaceutical composition thereof in a mass ratio of 4:1 to 240:1, preferably 10:1 to 180:1, 25:1 to 180:1, 30:1 to 180:1, or 60:1 to 180:1, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole.

9. The combined pharmaceutical composition according to claim 7, wherein the combined pharmaceutical composition is a formulation suitable for administration in a single treatment cycle (e.g., 28 days), and the formulation comprises: 1680-5040 mg, e.g., 3360-5040 mg, preferably 5040 mg, of the compound of formula (I) or the pharmaceutically acceptable salt thereof or a pharmaceutical composition thereof; and 70-140 mg, e.g., 70 mg, of letrozole or a pharmaceutical composition thereof, wherein the amount of the compound of formula (I) or the pharmaceutically acceptable salt thereof or the pharmaceutical composition thereof is based on the mass of the compound of formula (I), and the amount of letrozole or the pharmaceutical composition thereof is based on the mass of letrozole; or
the combined pharmaceutical composition is a formulation suitable for administration in a single treatment cycle (e.g., 28 days), and the formulation comprises: 1680-5040 mg, e.g., 3360-5040 mg, preferably 5040 mg, of the compound of formula (I) or the pharmaceutically acceptable salt thereof or a pharmaceutical composition thereof; and 28-140 mg, e.g., 28-56 mg, preferably 28 mg, of anastrozole or a pharmaceutical composition thereof, wherein the amount of the compound of formula (I) or the pharmaceutically acceptable salt thereof or the pharmaceutical composition thereof is based on the mass of the compound of formula (I), and the amount of anastrozole or the pharmaceutical composition thereof is based on the mass of anastrozole.

10. A method for treating or preventing breast cancer, comprising administering to an individual in need thereof a therapeutically effective amount of the combined pharmaceutical composition according to any one of claims 1 to 9.

11. Use of the combined pharmaceutical composition according to any one of claims 1 to 9 for preparing a medicament for treating or preventing breast cancer.

12. The combined pharmaceutical composition according to any one of claims 1 to 9 for use in treating or preventing breast cancer.

13. A method of administration, comprising administering to an individual in need thereof a therapeutically effective amount of the combined pharmaceutical composition according to any one of claims 1 to 9.

14. The method according to claim 10, the use according to claim 11, or the combined pharmaceutical composition for use according to claim 12, wherein the breast cancer is selected from the group consisting of:
HR-positive and HER2-negative breast cancer;
HR-positive and HER2-negative locally advanced and/or metastatic breast cancer;
HR-positive and HER2-negative locally advanced and/or metastatic breast cancer unable to receive radical surgery or radiotherapy; or
postmenopausal or premenopausal/perimenopausal HR-positive and HER2-negative locally advanced and/or metastatic breast cancer.

15. The method according to claim 10, the use according to claim 11, or the combined pharmaceutical composition for use according to claim 12, wherein 28 days are counted as one treatment cycle, the administration is performed once daily for 28 consecutive days, a total dose of the pharmaceutical composition comprising the compound of formula (I) or the pharmaceutically acceptable salt thereof administered per treatment cycle is 1680-5040 mg, and a total dose of the pharmaceutical composition comprising anastrozole administered per treatment cycle is 28-140 mg; or
28 days are counted as one treatment cycle, the administration is performed once daily for 28 consecutive days, a total dose of the pharmaceutical composition comprising the compound of formula (I) or the pharmaceutically acceptable salt thereof administered per treatment cycle is 1680-5040 mg, and a total dose of the pharmaceutical composition comprising letrozole administered per treatment cycle is 70-210 mg,
wherein the amount of the pharmaceutical composition comprising the compound of formula (I) or the pharmaceutically acceptable salt thereof is based on the mass of the compound of formula (I), and the amount of the pharmaceutical composition comprising anastrozole or the pharmaceutical composition comprising letrozole is based on the mass of anastrozole or letrozole, respectively.
